# EUROPÄISCHE PATENTSCHRIFT

(11) **EP 3 552 547 B1**
(45) Veröffentlichungstag und Bekanntmachung des Hinweises auf die Patenterteilung: **07.12.2022**
(21) Anmeldenummer: 18167300.5
(22) Anmeldetag: 13.04.2018
(51) Int. Cl.: A61B 6/00

(54) **VERFAHREN ZUR BEREITSTELLUNG EINER KONVERTIERUNGSINFORMATION ZU EINEM BILDDATENSATZ, RÖNTGENEINRICHTUNG, COMPUTERPROGRAMM UND ELEKTRONISCH LESBARER DATENTRÄGER**
METHOD FOR PROVIDING CONVERSION INFORMATION ABOUT AN IMAGING DATA SET, X-RAY DEVICE, COMPUTER PROGRAM AND ELECTRONICALLY READABLE DATA CARRIER
PROCÉDÉ DE FOURNITURE D'INFORMATIONS DE CONVERSION À UN ENSEMBLE DE DONNÉES D'IMAGE, DISPOSITIF À RAYONS X, PROGRAMME INFORMATIQUE ET SUPPORT DE DONNÉES LISIBLE ÉLECTRONIQUEMENT

(43) Veröffentlichungstag der Anmeldung: 16.10.2019
(73) Patentinhaber: Siemens Healthcare GmbH, 91052 Erlangen (DE)
(72) Erfinder: Ritter, André, 91054 Buckenhof (DE); Hofmann, Christian, 91052 Erlangen (DE)

(56) Entgegenhaltungen:
- WO-A1-2008/046498
- WO-A2-02/38045
- US-A1- 2014 236 488
- MATTHIAS WITT ET AL: "Optimization of the stopping-power-ratio to Hounsfield-value calibration curve in proton and heavy ion therapy", ZEITSCHRIFT FUER MEDIZINISCHE PHYSIK, Bd. 25, Nr. 3, 1. September 2015 (2015-09-01), Seiten 251-263, XP055512448, DE ISSN: 0939-3889, DOI: 10.1016/j.zemedi.2014.11.001

## Beschreibung

Die Erfindung betrifft ein Verfahren zur Bereitstellung einer eine Zuordnungsvorschrift wenigstens eines physikalischen Eigenschaftswerts eines Materials in einem Voxel zu einem Bildwert dieses Voxels in einem mit einer Röntgeneinrichtung aufgenommenen dreidimensionalen Bilddatensatz beschreibenden Konvertierungsinformation, wobei durch Vermessung eines wenigstens ein Kalibrierungsmaterial umfassenden Phantoms in der Röntgeneinrichtung ein Kalibrierungsdatensatz bestimmt wird, der zur Ermittlung der Zuordnungsvorschrift verwendet wird. Daneben betrifft die Erfindung eine Röntgeneinrichtung, ein Computerprogramm und einen elektronisch lesbaren Datenträger.

In verschiedenen Kontexten wurde vorgeschlagen, physikalische Eigenschaftswerte von Materialien, insbesondere im Bereich der Medizintechnik eines Patienten, aus dreidimensionalen Bilddatensätzen einer Röntgeneinrichtung, insbesondere einer Computertomographieeinrichtung, herzuleiten, nachdem die als Bildinformation in den dreidimensionalen Bilddatensätzen enthaltenen Schwächungskoeffizienten, insbesondere HU-Werte (HU - Hounsfield Units), in einem Zusammenhang mit solchen physikalischen Eigenschaftswerten stehen, insbesondere solchen, die in die Schwächungseigenschaften des Materials direkt eingehen. Insbesondere dann, wenn Hintergrundinformationen zu den Materialien im Aufnahmebereich ohnehin vorliegen, lassen sich mithin verschiedene physikalische Eigenschaftswerte schlussfolgern. Verschiedene Röntgeneinrichtungen weisen unterschiedliche Aufnahmeeigenschaften auf, wobei zudem die konkret erhaltenen Schwächungskoeffizienten in einem Bilddatensatz auch von Aufnahmeparametern zur Aufnahme des Bilddatensatzes sowie von Eigenschaften des aufgenommenen Objekts, insbesondere eines Patienten, abhängig sein können, so dass bekannte Verfahren zur Ermittlung einer Zuordnungsvorschrift, um aus Schwächungswerten für ein Voxel den physikalischen Eigenschaftswert des darin enthaltenen Materials zu erhalten, bereits vorgeschlagen wurde, zunächst einen Kalibrierungsdatensatz an einem Phantom aufzunehmen, welches definierte Materialien enthält, für die Informationen über die Abbildung der Schwächungseigenschaften erhalten werden. Nachdem für diese Materialien ebenso physikalische Informationen vorliegen, können als Konvertierungsinformation Zuordnungsvorschriften zu physikalischen Eigenschaftswerten abgeleitet werden.

Während derlei Konvertierungsinformationen in vielen Anwendungsgebieten, beispielsweise hinsichtlich eines Calciumscorings und/oder bei Hybridmodalitäten eingesetzt werden können, ist als ein wesentliches Anwendungsgebiet solcher Zuordnungsvorschriften die Strahlentherapie bzw. konkret deren Planung zu nennen. Insbesondere im Hinblick auf die Nutzung von Partikelstrahlung, beispielsweise Protonenbestrahlung, müssen für eine Behandlung am korrekten Ort physikalische Eigenschaftswerte berücksichtigt werden, die sich aus Röntgenaufnahmen, insbesondere Computertomographieaufnahmen, ableiten lassen. Ein beispielhafter Vergleich unterschiedlicher Konvertierungsansätze bezüglich der Partikeltherapie findet sich in einem Artikel von M. Witt et al. "Optimization of the stopping-power-ratio to Hounsfield-value calibration curve in proton and heavy ion therapy", Z. Med. Phys. 25 (3), Seiten 251 bis 263, 2015, wo es konkret um die Ermittlung des Bremsvermögens als physikalischen Eigenschaftswert aus HU-Werten geht. Die hierin ebenso diskutierte stöchiometrische Kalibrierung ist grundlegend in einem Artikel von U. Schneider et al., "The calibration of CT Hounsfield units for radiotherapy treatment planning", Phys. Med. Biol. 41 (1996), Seiten 111 bis 124, beschrieben.

Bei einer Anwendung der physikalischen Eigenschaftswerte in der Bestrahlungsplanung wird gewöhnlicher Weise eine Dosisverteilung ermittelt, die aufgrund der Bestrahlung zu erwarten ist. Die aufgrund der Konvertierungsinformation ermittelbare Verteilung des physikalischen Eigenschaftswerts im Patienten, wobei der physikalische Eigenschaftswert beispielsweise die Elektronendichte, die Massendichte und/oder das Bremsvermögen wiedergegeben kann, wird genutzt, um die Energiedeposition durch die Strahlung im Patienten zu beschreiben und entsprechend bezüglich des Therapieziels zu optimieren. Üblicherweise werden als Bilddatensätze Computertomographieaufnahmen des Patienten in der Position gewonnen, die auch zur Bestrahlung verwendet werden kann.

Dabei ist es im Stand der Technik bekannt, die Umwandlung der Bildwerte, konkret HU-Werte, in den Bilddatensätzen zu den physikalischen Eigenschaftswerten durch eine sogenannte Konversionstabelle als Konvertierungsinformation durchzuführen, wobei diese Konversionstabelle durch eine Computertomographiemessung an einem Phantom, wie eingangs bereits erläutert, erzeugt wird. Ein geeignetes Phantom enthält meist mehrere Kalibrierungsmaterialien, die die Bandbreite der Körpergewebe in Bezug auf den physikalischen Eigenschaftswert und den Bildwert simulieren. Die sich aus der Kalibrierungsmessung ergebende Zuordnung zwischen physikalischer Größe und Bildwert kann dabei direkt als Zuordnungsvorschrift übernommen werden.

Die bereits erwähnte stöchiometrische Kalibrierung ermöglicht es, höhere Genauigkeiten zu erreichen, wobei hier die Werte der Phantom-Messung nicht unmittelbar übernommen werden. Stattdessen wird ein analytisches Modell der sich ergebenden Bildwerte in Abhängigkeit der Dichte und stöchiometrischen Zusammensetzung eines Materials verwendet. Das Modell enthält freie Parameter, die in Abhängigkeit der Kalibrierungsmessung an dem Phantom festgelegt werden. Die im Phantom verwendeten Kalibrierungsmaterialien unterliegen dabei nicht der Einschränkung, möglichst gewebeähnlich zu sein, sondern es können Kalibrierungsmaterialien eingesetzt werden, die zwar eine gewisse Ähnlichkeit zu im Körper vorkommenden Materialien bezüglich der stöchiometrischen Zusammensetzung aufweisen, deren Zusammensetzung aber exakt bekannt ist und mit dem mathematischen Modell gut wiedergegeben werden kann. Sind die freien Modellparameter des Modells bekannt, kann anhand der bekannten stöchiometrischen Zusammensetzung echter menschlicher Gewebe wiederum eine Konversionstabelle bzw. eine sonstige Zuordnungsvorschrift erzeugt und beispielsweise in einer Planungseinrichtung für die Planung der Strahlentherapie hinterlegt werden.

Im Stand der Technik werden die Zuordnungsvorschriften, mithin die Konvertierungsinformationen, üblicherweise unmittelbar in der Planungseinrichtung, also einem Planungssystem, hinterlegt, mit der bzw. dem die Bestrahlungsplanung durchgeführt werden soll. Hierbei existiert insbesondere das Problem, dass die HU-Werte nicht universell für alle Röntgeneinrichtungen und alle verwendbaren Röntgenspektren sind. Eine Hinterlegung von einer großen Vielzahl an Konvertierungsinformationen für unterschiedliche Geräte, Spektren und dergleichen in der Planungseinrichtung wäre jedoch mit einem zu großen Aufwand verbunden. Dabei existiert zudem das Problem, dass auch der Patient selber in gewissem Maße "filternd" wirkt, was sich auf das durch den Röntgendetektor der Röntgeneinrichtung empfangene Spektrum und somit die HU-Werte auswirken kann. Auch können sich Eigenschaften der Röntgeneinrichtungen mit deren Alter ändern. Gerade bei der Partikelbestrahlung, beispielsweise der Verwendung von Protonen bzw. sonstigen Ionen, wo eine harte Abbruchkante bei der Energiedeposition vorliegt, ist eine äußerst genaue Vorausberechnung der Dosisverteilung wesentlich, um beispielsweise einen Tumor möglichst exakt behandeln zu können, so dass auch derartige kleinere Unterschiede wesentlich sein können.

Der Erfindung liegt daher die Aufgabe zugrunde, eine Möglichkeit zur einfachen Bereitstellung hochgenauer, auf den Patienten und/oder die verwendete Röntgeneinrichtung abgestellter Konvertierungsinformationen, insbesondere für die Bestrahlungsplanung, bereitzustellen.

Zur Lösung dieser Aufgabe ist bei einem Verfahren der eingangs genannten Art erfindungsgemäß vorgesehen, dass der Bilddatensatz mit einem auf einem Röntgendetektor der Röntgeneinrichtung vermessenen Empfangsspektrum aufgenommen wird, das durch wenigstens einen Spektrumsparameter beschrieben wird, wobei zur Ermittlung der Zuordnungsvorschrift in Abhängigkeit von dem Spektrumsparameter aus dem verschiedene Empfangsspektren beschreibend gemessenen Kalibrierungsdatensatz abgeleitete Kalibrierungsdaten verwendet werden.

Erfindungsgemäß wird mithin vorgeschlagen, auf das von dem Röntgendetektor empfangene Empfangsspektrum abzustellen, welches durch verschiedene Spektrumsparameter beschrieben werden kann, die üblicherweise auch bei oder durch die Aufnahme des Bilddatensatzes bekannt sind bzw. zumindest ableitbar sind. Liegt nun ein Kalibrierungsdatensatz vor, der ebenso verschiedene Empfangsspektren beschreibt und somit eine Auswahl bzw. Ableitung geeigneter Kalibrierungsdaten erlaubt, lassen sich hochgenaue, auf den speziellen Vorgang der Aufnahme des Bilddatensatzes und/oder auf den speziellen Patienten abgestellte Konvertierungsinformationen erzeugen, welche mithin zu einer genaueren Bestimmung des wenigstens einen physikalischen Eigenschaftswertes führen, so dass beispielsweise eine Bestrahlungsplanung, eine Scoring-Applikation und dergleichen verbesserte Informationen über den Patienten liefern können, zumindest dessen in dem Bilddatensatz enthaltenen Aufnahmebereich.

Wie im Stand der Technik grundsätzlich bekannt kann die Zuordnungsvorschrift eine Look-Up-Tabelle (Konversionstabelle) und/oder eine Abbildungsfunktion sein bzw. umfassen und/oder die Konvertierungsinformation kann freie Modellparameter eines zur Konvertierung verwendeten Modells, mithin die Look-Up-Tabelle oder die Abbildungsfunktion parametrisierende Größen, umfassen, wie dies im Stand der Technik grundsätzlich bekannt ist. Bei der Röntgeneinrichtung handelt es sich insbesondere um eine Computertomographieeinrichtung, welche besonders genaue dreidimensionale Informationen, mithin Schwächungskoeffizienten (HU-Werte) als Bildwerte für im dreidimensionalen Raum definierte Voxel innerhalb eines Patienten mit hoher Qualität liefert. Aufgrund der Konvertierungsinformation können die entsprechend insbesondere als HU-Werte vorliegenden Bildwerte in physikalische Eigenschaftswerte in diesem Voxel umgerechnet werden, beispielsweise eine Elektronendichte, eine Massendichte und/oder ein Bremsvermögen (stopping power).

An der Röntgeneinrichtung wird ein Kalibrierungsverfahren implementiert, welches ein vordefiniertes, technisches Phantom nutzt, das geeignet ist, die benötigten Kalibrierungsdaten zu bestimmen. Hierzu kann das Phantom bevorzugt mehrere, beispielsweise fünf, geeignete Kalibrierungsmaterialien enthalten, welche bei der stöchiometrischen Kalibrierung beispielsweise Teflon und PMMA umfassen können. Die Kalibrierungsmaterialien liegen bevorzugt zylinderförmig vor und sind in einen zylinderförmigen Körper, bestehend aus einem wasseräquivalenten Phantommaterial, eingebettet. Durch Messungen mit der Röntgeneinrichtung an diesem Phantom können, wie noch genauer erläutert werden wird, für unterschiedliche Spektrumsparameter Kalibrierungsdaten als ein Kalibrierungsdatensatz aufgenommen werden, wobei der (jeweils aktuelle) Kalibrierungsdatensatz bevorzugt dauerhaft an der Röntgeneinrichtung vorgehalten wird.

Wird nun ein Bilddatensatz eines Aufnahmebereichs eines Patienten mit speziellen Spektrumsparametern angefertigt, kann der Kalibrierungsdatensatz genutzt werden, um nach Auswahl bzw. Ableitung der geeigneten Kalibrierungsdaten gemäß der Spektrumsparameter, wie mit Vorliegen von Kalibrierungsdaten grundsätzlich bekannt, die geeigneten Konvertierungsinformationen herzuleiten. Dabei sei darauf hingewiesen, dass die Anwendung der vorliegenden Erfindung auf eine stöchiometrische Kalibrierung bevorzugt ist, jedoch jede Art der Kalibrierung eingesetzt werden kann, bei der auf Grundlage von Kalibrierungsdaten Konvertierungsinformationen abgeleitet werden können, welche eine Zuordnungsvorschrift enthalten bzw. parametrieren, um physikalische Zielgrößen, also Eigenschaftswerte, einem Bildwert des Bilddatensatzes zuzuordnen.

Insgesamt wird durch diesen Ansatz grundsätzlich eine Arbeitserleichterung geschaffen, nachdem es nicht mehr nötig ist, Kalibrierungsdaten für jeden einzelnen Untersuchungsvorgang eines Patienten zu ermitteln, sondern geeignete Kalibrierungsdaten aus einem umfassenderen Kalibrierungsdatensatz aufgrund der Spektrumsparameter eines aktuellen Untersuchungsvorgangs abgeleitet werden können. Insbesondere ist hierbei eine gewisse Automatisierung möglich, nachdem bei Kenntnis der Spektrumsparameter eine manuelle Auswahl bzw. Pflege von Konvertierungsinformationen entfallen kann, somit auch eine mögliche Fehlerquelle. Mit besonderem Vorteil aber ist es möglich, auch patientenspezifische Konvertierungsinformationen zu erzeugen.

In diesem Zusammenhang sieht eine besonders vorteilhafte Ausgestaltung der vorliegenden Erfindung vor, dass als Spektrumsparameter ein eine Ausdehnung des Patienten insbesondere schichtspezifisch beschreibender Ausdehnungsparameter verwendet wird und als das Phantom ein ein wasseräquivalent schwächendes Phantommaterial um das wenigstens eine Kalibrierungsmaterial aufweisendes Phantom verwendet wird. Das bedeutet, im Rahmen der vorliegenden Erfindung ist es besonders vorteilhaft möglich, die filternde Wirkung des Patienten auf das Empfangsspektrum zu berücksichtigen, indem einer der Spektrumsparameter ein Ausdehnungsparameter des Patienten ist, der zu einer entsprechenden Auswahl von Kalibrierungsparametern führt. Letztlich wird hierbei berücksichtigt, dass die Röntgenstrahlung zur Aufnahme des Bilddatensatzes den Patienten durchqueren muss, mithin eine wasseräquivalente Durchstrahlungslänge zu berücksichtigen ist, die durch die Ausdehnung des Patienten beschrieben wird und im Phantom durch ein entsprechendes, wasseräquivalentes Phantommaterial abgebildet werden kann. Mit besonderem Vorteil kann dabei für jede Schicht, die durch den Bilddatensatz abgedeckt ist, ein eigener Satz von Konvertierungsinformationen ermittelt werden, da die Ausdehnung des Patienten von Schicht zu Schicht (bzw. Schnittbild zu Schnittbild) variieren kann. Durch Berücksichtigung von das Empfangsspektrum beeinflussenden Patienteneigenschaften wird hiermit eine noch genauere Konvertierung, insbesondere schichtspezifisch, ermöglicht.

Dabei ist es besonders zweckmäßig, wenn als das Phantom ein Phantom mit für jedes Kalibrierungsmaterial mehreren geometrischen Ausdehnungen, insbesondere Durchmessern, des das Kalibrierungsmaterial umgebenden Phantommaterials verwendet wird. Das bedeutet, um unterschiedliche wasseräquivalente Durchstrahlungslängen auch für das Phantom wiedergeben zu können, kann dieses eine geeignete geometrische Ausgestaltung aufweisen, in der mehrere unterschiedliche Durchstrahlungslängen des Phantommaterials gegeben sind. Hierzu bietet es sich insbesondere an, ein zylindrisches, gestufte Außendurchmesser aufweisendes Phantom zu verwenden. Auf diese Weise werden mithin für unterschiedliche Durchstrahlungslängen des Phantommaterials, welches sich bezüglich der Röntgenaufnahme wasseräquivalent verhält, Kalibrierungsdaten erhalten, wobei es beispielsweise denkbar ist, für dazwischenliegende Ausdehnungsparameter eines Patienten Interpolationen in den Kalibrierungsdaten durchzuführen. Mithin kann bereits in dem Kalibrierungsdatensatz die Abhängigkeit des Bildwerts des Bilddatensatzes von der effektiven Wasserdurchstrahlungslänge charakterisiert werden.

Der Ausdehnungsparameter kann bevorzugt einen mittleren Durchmesser des Patienten umfassen, der zur Ableitung der Kalibrierungsdaten aus dem Kalibrierungsdatensatz als Durchmesser des Phantoms verwendet wird. Ein mittlerer, effektiver Durchmesser des Patienten hat sich dabei als äußerst genaue Abschätzung hinsichtlich der Filterwirkung über die gesamten, in den Bilddatensatz eingehenden Projektionsdaten erwiesen. Während es grundsätzlich denkbar wäre, auch eine Annahme des Patienten als Ellipse und eine entsprechende elliptische Ausgestaltung eines Phantoms heranzuziehen, ist dies jedoch mit einem deutlich größeren Aufwand verbunden, welcher nur eine geringere Genauigkeitsverbesserung mit sich bringt. Daher wird erfindungsgemäß vorgeschlagen, einen mittleren, mithin effektiven, Durchmesser des Patienten zu verwenden. Liegen, was bevorzugt ist, Kalibrierungsdaten für verschiedene Durchmessers des Phantoms vor, kann eine entsprechende Auswahl von Kalibrierungsdaten aus dem Kalibrierungsdatensatz bzw. durch Interpolation eine Berechnung von geeigneten Kalibrierungsdaten erfolgen.

Der Ausdehnungsparameter kann zweckmäßigerweise aus dem Bilddatensatz selber und/oder aus einem zur Planung der Aufnahme des Bilddatensatzes aufgenommenen Topogramm ermittelt werden. Das bedeutet, aktuelle, im Rahmen des Untersuchungsvorgangs ermittelte Informationen werden herangezogen, um mit besonderem Vorteil schichtspezifisch den Ausdehnungsparameter, beispielweise als mittleren Durchmesser, zu bestimmen. Dies ist durch eine einfache Segmentierung für die als Teil des Bilddatensatzes insbesondere entstehenden Schnittbilder in einem konkreten Beispiel problemlos möglich.

Dabei sei darauf hingewiesen, dass es in weniger bevorzugten Ausführungsbeispielen grundsätzlich auch denkbar ist, den Ausdehnungsparameter abzuschätzen, beispielsweise aus Patienteninformationen wie Geschlecht, Größe, Gewicht, BMI, Aufnahmebereich und dergleichen.

Wie bereits erwähnt, ist es im Rahmen der vorliegenden Erfindung besonders bevorzugt, wenn bei für jede Schicht des Bilddatensatzes vorliegendem Ausdehnungsparameter die Konvertierungsinformation schichtspezifisch ermittelt wird. Das bedeutet, für jede Schicht und jeden physikalischen Eigenschaftswert enthält die Konvertierungsinformation eine Zuordnungsvorschrift bzw. die Zuordnungsvorschrift parametrierende Angaben, beispielsweise Modellparameter. Mit besonders hoher Genauigkeit und Qualität kann die Zuordnungsvorschrift dann, abhängig von den Eigenschaften des mittleren detektierten Empfangsspektrums und damit abhängig von der effektiven mittleren wasseräquivalenten Durchstrahlungslänge, aus den Bildwerten des Bilddatensatzes, insbesondere also den HU-Werten, den wenigstens einen physikalischen Eigenschaftswert bestimmen. Dies wird durch patientenspezifische Konvertierungsinformationen erreicht.

Im Rahmen der vorliegenden Erfindung können selbstverständlich auch andere Spektrumsparameter, die auf die Röntgeneinrichtung selbst bezogen sein können, herangezogen werden. So sieht eine bevorzugte Weiterbildung vor, dass als Spektrumsparameter wenigstens ein das von einer Röntgenquelle erzeugte Sendespektrum beschreibender Erzeugungsparameter, insbesondere eine Röhrenspannung, und/oder wenigstens ein wenigstens ein verwendetes Filter beschreibender Filterparameter verwendet wird, wobei der Kalibrierungsdatensatz für mehrere unterschiedliche Erzeugungsparameter bzw. Filterparameter vermessen wird. Somit liegen für die unterschiedlichen Erzeugungsparameter und Filterparameter Kalibrierungsdaten im Kalibrierungsdatensatz vor oder können aus diesen, insbesondere durch Interpolation, zumindest abgeleitet werden. Auf diese Weise werden die Eigenschaften der Röntgeneinrichtung auch für mehrere diesbezügliche Aufnahmeparameter, beispielsweise unterschiedliche verwendbare Sendespektren bzw. unterschiedliche verwendbare Filtereinstellungen, ermittelt und zur weiteren Erhöhung der Qualität der mittels der Konvertierungsinformationen bestimmbaren physikalischen Eigenschaftswerte genutzt.

Wie bereits angedeutet wurde, sieht eine vorteilhafte Ausgestaltung des erfindungsgemäßen Verfahrens vor, dass für wenigstens einen Spektrumsparameter des Bilddatensatzes, zu dem keine Kalibrierungsdaten im Kalibrierungsdatensatz vorliegen, passende Kalibrierungsdaten durch Interpolation abgeleitet werden können. Das bedeutet, es wird eine geeignete Anzahl an Stützstellen für jeden Spektrumsparameter festgelegt, beispielsweise durch eine gestufte Ausbildung des Phantoms für mehrere vorgegebene Durchmesser des Phantoms und/oder mehrere Messvorgänge bei unterschiedlichen Erzeugungsparametern und/oder Filterparametern, wobei für weitere konkrete Spektrumsparameter durch Interpolation aus dem Kalibrierungsdatensatz an diesen Stützstellen entsprechende Kalibrierungsdaten abgeleitet und zur Ermittlung der Konvertierungsinformation herangezogen werden können. Auf diese Weise ist eine noch genauere Bestimmung der physikalischen Eigenschaftswerte als bei Verwendung der Kalibrierungsdaten der nächstgelegenen Stützstelle gegeben.

Im Rahmen des erfindungsgemäßen Verfahrens wird mit besonderem Vorteil eine stöchiometrische Kalibrierung verwendet, wobei die freien Modellparameter eines zur Ermittlung der Zuordnungsvorschrift verwendeten Stöchiometriemodells durch die Kalibrierungsdaten abhängig von dem wenigstens einen Spektrumsparameter beschrieben werden. Zur grundsätzlichen Beschreibung der stöchiometrischen Kalibrierung sei auf den eingangs zitierten Artikel von Uwe Schneider et al. verwiesen. Als Konvertierungsinformation können in diesem Zusammenhang also insbesondere freie Modellparameter in Abhängigkeit der Spektrumsparameter aus den Kalibrierungsdaten ermittelt werden, aus denen beispielsweise ein entsprechender Zusammenhang zwischen den freien Modellparametern und dem Spektrumsparameter abgeleitet werden kann.

In einer bevorzugten Weiterbildung in diesem Kontext kann vorgesehen sein, dass die Konvertierungsinformation für mehrere stöchiometrisch beschriebene, innerhalb eines Patienten auftretende Patientenmaterialien ermittelt wird, wobei die Zahl und/oder Art der Patientenmaterialien, für die Konvertierungsinformation bestimmt wird, aufgrund einer Patienteninformation beschränkt und/oder definiert wird. Das bedeutet, die Auswahl der Konvertierungsstützstellen/Patientenmaterialien muss nicht zwangsläufig fest vorgegeben sein, sondern kann auch durch eine Patienteninformation beeinflusst werden. Beispielsweise ist es denkbar, dass die Patienteninformation einen Aufnahmebereich des Bilddatensatzes und/oder ein verwendetes Aufnahmeprotokoll und/oder das Alter und/oder das Geschlecht des Patienten umfasst. Die Auswahl kann auch von anderen physiologischen/anatomischen Merkmalen beeinflusst werden. Dabei ist es konkret denkbar, dass die Patienteninformation wenigstens teilweise automatisch durch die Röntgeneinrichtung und/oder wenigstens teilweise manuell durch eine Benutzereingabe ermittelt wird. Ist beispielsweise die Röntgeneinrichtung in der Lage, aufgenommene Körperregionen zu erkennen und/oder kann die Patienteninformation auf anderem Wege, beispielsweise über eine Verbindung zu einem Informationssystem, erhalten werden, kann zumindest ein Teil der Patienteninformation automatisch ermittelt werden. Zweckmäßig kann es jedoch auch sein, wenn ein Bediener in der Lage ist, Einfluss auf die entsprechende Auswahl, insbesondere Beschränkung, auf bestimmte Patientenmaterialien zu nehmen. In einem Ausführungsbeispiel ist es insbesondere denkbar, dass der Bediener als Benutzereingabe die Dichte und die stöchiometrische Zusammensetzung der Patientenmaterialien, beispielsweise in einem geeigneten Benutzerinterface, direkt konfigurieren kann.

Diese Ausgestaltung erlaubt also letztlich das Nutzen von Vorabwissen über den Patienten bzw. den Aufnahmebereich des Bilddatensatzes. Ist als Patienteninformation beispielsweise bekannt, dass im Brustbereich eine Aufnahme einer Frau durchgeführt wurde, sollte Brustdrüsengewebe in den Konvertierungsinformationen abgedeckt sein, während dies bei einem Mann unnötig ist. Entsprechend können im Brustbereich Gewebearten, die nur im Bauchbereich auftreten, bei den Konvertierungsinformationen ausgespart werden, welche somit insgesamt "maßgeschneidert" für den aktuellen Anwendungsfall, mithin den Bilddatensatz und dem Patienten, geliefert werden. Dabei ist die entsprechende Ausgestaltung, in der die Zahl abzudeckender Patientenmaterialien reduziert ist, besonders im Fall von Look-Up-Tabellen und/oder sonstigen Zuordnungsvorschriften zweckmäßig, die für deren Abdeckung die Menge der Konvertierungsinformationen deutlich erhöhen würden. Mit der hier vorgeschlagenen Berücksichtigung von Patienteninformationen kann jedoch die Datenmenge an Konvertierungsinformationen gering gehalten werden, was insbesondere bei der Übermittlung von Konvertierungsinformationen, beispielsweise an eine Planungseinrichtung, zweckmäßig sein kann.

Eine besonders vorteilhafte Ausgestaltung der vorliegenden Erfindung sieht vor, dass die Konvertierungsinformation seitens der Röntgeneinrichtung ermittelt, und gemeinsam mit dem Bilddatensatz an eine den Bilddatensatz weiter auswertende Auswertungseinrichtung übermittelt. Nachdem die ermittelten Konvertierungsinformationen nicht nur für die Röntgeneinrichtung spezifisch sind, sondern auch für den speziellen Bilddatensatz/Patienten, werden sie zweckmäßigerweise bereits seitens der Röntgeneinrichtung generiert und gemeinsam mit dem Bilddatensatz an eine nachgeschaltete Einrichtung, insbesondere eine Planungseinrichtung, ausgeliefert. Nachdem es aufgrund des Kalibrierungsdatensatzes auch möglich ist, seitens der Röntgeneinrichtung die Konvertierungsinformation automatisch zu erstellen, entfallen manuelle Aufwände zum Erstellen und Pflegen von Konvertierungsinformationen, insbesondere seitens der Planungseinrichtung, nachdem eine erweiterte Nutzung vorhandener Möglichkeiten der Röntgeneinrichtung erfolgt. Eine mögliche manuelle Anwahl einer Konvertierungsinformation in der Auswertungseinrichtung, insbesondere Planungseinrichtung, entfällt, so dass auch eine mögliche Fehlerquelle entfällt. Damit wird eine deutliche Arbeitserleichterung bereitgestellt.

Dabei sei in diesem Kontext insbesondere angemerkt, dass es zwar grundsätzlich denkbar wäre, neben dem Bilddatensatz auch bereits die physikalischen Eigenschaftswerte als Eigenschaftsdatensatz seitens der Röntgeneinrichtung zu erstellen und an die Auswerteeinrichtung zu ermitteln. Dies ist jedoch weniger bevorzugt, da Bediener dazu tendieren, einen Bilddatensatz zum Betrachten zu erhalten und weitere Datensätze, hier den Eigenschaftsdatensatz, die keinen Betrachtungsnutzen aufweisen, eher ablehnen. Zudem wird die Menge der zu übertragenden Daten deutlich reduziert und die physikalischen Eigenschaftswerte können bei Bedarf vor Ort in der Auswertungseinrichtung, insbesondere Planungseinrichtung, anhand der Konvertierungsinformation ermitteln werden. Dies ist insbesondere dann zweckmäßig wenn die physikalischen Eigenschaftswerte nur in bestimmten Bereichen des Bilddatensatzes überhaupt benötigt werden oder dergleichen.

Es sei ferner noch allgemein darauf hingewiesen, dass die Konvertierungsinformationen bevorzugt auch eine Information darüber umfassen, für welche physikalischen Eigenschaftswerte die entsprechend enthaltenen Zuordnungsvorschriften/Modellparameter geeignet sind.

Vorzugsweise kann die Konvertierungsinformation dem Bilddatensatz als Metadaten hinzugefügt werden, insbesondere als DICOM-Metadaten, und/oder der Bilddatensatz kann durch Auswertung zur Kontrastoptimierung bezüglich einer Darstellung bearbeitet übermittelt werden. Besonders vorteilhaft lassen sich die Konvertierungsinformationen als Metadaten dem Bilddatensatz hinzufügen, bilden mithin seitens der Auswerteeinrichtung einen integralen Teil des Bilddatensatzes bzw. des entsprechenden Datenobjekts, so dass ein unmittelbarer Zugriff auf eine definierte Art geschieht und die physikalischen Eigenschaftswerte besonders einfach ermittelt werden können. Im DICOM-Format ist ohnehin ein Freiraum für benutzerseitig definierbare Metadaten vorgesehen, welcher zweckmäßig für die Konvertierungsinformation genutzt werden kann.

Vorteilhaft ist es ferner auch, wenn der Bilddatensatz zu Darstellungszwecken kontrastoptimiert ist. Damit ist ein weiterer, deutlicher Vorteil gegenüber beispielsweise einem Vorgehen gegeben, welches unmittelbar Schichtbilder der physikalischen Eigenschaftswerte an die Auswertungseinrichtung liefert. Beispielsweise bei einer Planungseinrichtung als Auswertungseinrichtung ist es in der erfindungsgemäßen Weiterbildung dagegen problemlos möglich, den Bilddatensatz weiterhin zu Darstellungszwecken zu nutzen, beispielsweise um die Lage des Behandlungsgebiets, beispielsweise eines Tumors, aus den Bildwerten des Bilddatensatzes zu schließen und entsprechend in die Planung einzubeziehen. Die physikalischen Eigenschaftswerte können dann bei Bedarf, im Beispiel bei der Dosisberechnung, aufgrund der mitgelieferten Konvertierungsinformation schnell und einfach ermittelt werden. Es hat sich gezeigt, dass bei der Umrechnung von Bilddatensätzen zu physikalischen Eigenschaftswerten Kontrastverluste auftreten können, nachdem beispielsweise eine Elektronendichte eine geringere Kontrastdynamik als gängige Schwächungskoeffizienten aufweist. Nachdem der für die Darstellung optimierte Bilddatensatz gemeinsam mit dem Konvertierungsinformationen übermittelt wird, lassen sich die Vorteile beider Datenformen in kompakter Art vereint bereitstellen.

Dabei sei an dieser Stelle noch angemerkt, dass nicht zwangsläufig die Ermittlung der Konvertierungsinformation in der Röntgeneinrichtung als solche erfolgen muss, sondern auch beispielsweise eine Zwischeneinrichtung genutzt werden kann, an die der Bilddatensatz übermittelt wird und die Zugriff auf den Kalibrierungsdatensatz hat, so dass sie die Konvertierungsinformation ermittelt und gegebenenfalls als Metadaten dem Bilddatensatz hinzufügen kann, um diesen an die Auswertungseinrichtung weiterzuleiten. Auch verteilte Ermittlungen der Konvertierungsinformationen sind denkbar. In diesem Kontext sei noch allgemein darauf hingewiesen, dass auf die Röntgeneinrichtung bezogene Spektrumsparameter, insbesondere also Erzeugungsparameter und/oder Filterparameter, bei Verwendung des DICOM-Formates meist ohnehin innerhalb des entsprechenden Datenobjekts des Bilddatensatzes gespeichert sind und auch an anderen Orten mithin vorliegen.

Besonders vorteilhaft lässt sich das erfindungsgemäße Verfahren bei der Strahlentherapieplanung, insbesondere einer Partikeltherapieplanung, einsetzen. So kann vorgesehen sein, dass die Konvertierungsinformationen für eine Strahlentherapieplanung verwendet werden, insbesondere die Auswertungseinrichtung eine Planungseinrichtung eines Planungssystems ist. Gerade bei der Partikeltherapie, insbesondere also der Bestrahlung mit Protonen oder schweren Ionen, ist eine hohe Genauigkeit der Bestimmung der physikalischen Eigenschaftswerte, beispielsweise eines Bremsvermögens, einer Elektronendichte und/oder einer Massendichte, wichtig, um den Behandlungserfolg möglichst weitgehend sicherstellen zu können.

Zweckmäßigerweise kann die Vermessung des Kalibrierungsdatensatzes zyklisch, insbesondere in Wartungsintervallen, wiederholt werden. Insbesondere als Teil regelmäßiger Qualitätssicherungsmaßnahmen an der Röntgeneinrichtung, beispielsweise zwischen täglich und jährlich, kann die Kalibrierungsmessung an dem Phantom vorgenommen werden, wonach der jeweils aktuelle Kalibrierungsdatensatz gespeichert wird. So liegen auch bei Veränderungen an der Röntgeneinrichtung immer geeignete Kalibrierungsdaten im Kalibrierungsdatensatz vor.

Vorzugsweise kann abgesehen von der Einbringung des Phantoms durch einen Bediener die Vermessung des Kalibrierungsdatensatzes vollständig automatisch nach einem Kalibrierungsprogramm von der Röntgeneinrichtung durchgeführt werden. Beispielsweise kann also das Phantom von dem Bediener auf und/oder an einer Patientenliege der Röntgeneinrichtung montiert werden. Daraufhin kann die Röntgeneinrichtung das Phantom automatisch positionieren und in einem weiteren Schritt geeignete Röntgenmessungen an dem Phantom durchführen, insbesondere bei unterschiedlichen Erzeugungsparametern und/oder Filterparametern, wozu das Kalibrierungsprogramm genutzt wird. Die Messergebnisse werden dann ausgewertet, um die Bildwerte aller im Phantom befindlichen Kalibrierungsmaterialien spektrumsabhängig, was sich auch auf unterschiedliche Durchmesser des Phantoms beziehen kann, zu bestimmen. Hieraus ergibt sich der Kalibrierungsdatensatz. Besonders bevorzugt wird insbesondere wenigstens der jeweils aktuelle Kalibrierungsdatensatz in einer Datenbank der Röntgeneinrichtung abgespeichert. Nachdem der Kalibrierungsdatensatz für verschiedene Spektrumsparameter gültig ist, kann er mithin auf eine Vielzahl von Messungen mit der Röntgeneinrichtung angewandt werden, so dass nicht vor jeder Aufnahme eines Bilddatensatzes zunächst eine Messung an dem Phantom erfolgen muss.

Es sei noch darauf hingewiesen, dass das erfindungsgemäße Verfahren selbstverständlich auch auf Mehrenergie-Computertomographie bzw. spektrale Bildgebung übertragbar ist. Die entsprechenden Zuordnungsvorschriften weisen in diesem Fall wenigstens zwei Bildwerten (der unterschiedlichen Energien/Sendespektren) einen physikalischen Eigenschaftswert zu.

Neben dem Verfahren betrifft die Erfindung auch eine Röntgeneinrichtung, insbesondere eine Computertomographieeinrichtung, aufweisend eine einen Röntgendetektor und eine Röntgenquelle aufweisende Aufnahmeanordnung und eine zur Durchführung des erfindungsgemäßen Verfahrens ausgebildete Steuereinrichtung. Sämtliche Ausführungen bezüglich des erfindungsgemäßen Verfahrens lassen sich analog auf die erfindungsgemäße Röntgeneinrichtung übertragen, mit welcher mithin ebenso die bereits genannten Vorteile erhalten werden können. Die Steuereinrichtung kann wenigstens einen Prozessor und wenigstens eine Speichermittel aufweisen, durch welches insbesondere eine Datenbank zur Speicherung des Kalibrierungsdatensatzes realisiert ist. Neben einer Aufnahmeeinheit, die, wie grundsätzlich bekannt, den Aufnahme- bzw. Messbetrieb der Röntgeneinrichtung steuert und die auch zur Aufnahme des Kalibrierungsdatensatzes und des Bilddatensatzes herangezogen werden kann, kann die Steuereinrichtung insbesondere eine Konvertierungsinformationsermittlungseinheit aufweisen, um die Spektrumsparameter zu nutzen und die geeignete Konvertierungsinformationen zu bestimmen. Auch weitere Funktionseinheiten gemäß beschriebener möglicher Schritte des erfindungsgemäßen Verfahrens können vorgesehen sein.

Ein erfindungsgemäßes Computerprogramm ist beispielsweise direkt in einen Speicher einer Recheneinrichtung, insbesondere einer Steuereinrichtung einer Röntgeneinrichtung und/oder einer Zwischeneinrichtung, ladbar und weist Programmmittel auf, um die Schritte eines erfindungsgemäßen Verfahrens auszuführen, wenn das Computerprogramm in der Recheneinrichtung ausgeführt wird. Das Computerprogramm kann auf einem erfindungsgemäßen elektronisch lesbaren Datenträger gespeichert sein, welcher mithin darauf gespeicherte elektronisch lesbare Steuerinformationen umfasst, welche zumindest ein erfindungsgemäßes Computerprogramm umfassen, und derart ausgestaltet sind, dass sie bei Verwendung des Datenträgers in einer Recheneinrichtung ein erfindungsgemäßes Verfahren durchführen. Bei dem Datenträger kann es sich um einen nichttransienten Datenträger, insbesondere eine CD-ROM, handeln.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: einen Ablaufplan eines Ausführungsbeispiels des erfindungsgemäßen Verfahrens in einer Kalibrierungsphase,
- Fig. 2: ein verwendbares Phantom,
- Fig. 3: einen Ablaufplan eines Ausführungsbeispiels des erfindungsgemäßen Verfahrens in einer Konvertierungsinformationsbestimmungsphase,
- Fig. 4: eine mögliche Abhängigkeit eines Modellparameters von einem Spektrumsparameter,
- Fig. 5: eine erfindungsgemäße Röntgeneinrichtung, und
- Fig. 6: eine mögliche Ausgestaltung eines Planungssystems für Strahlentherapie.

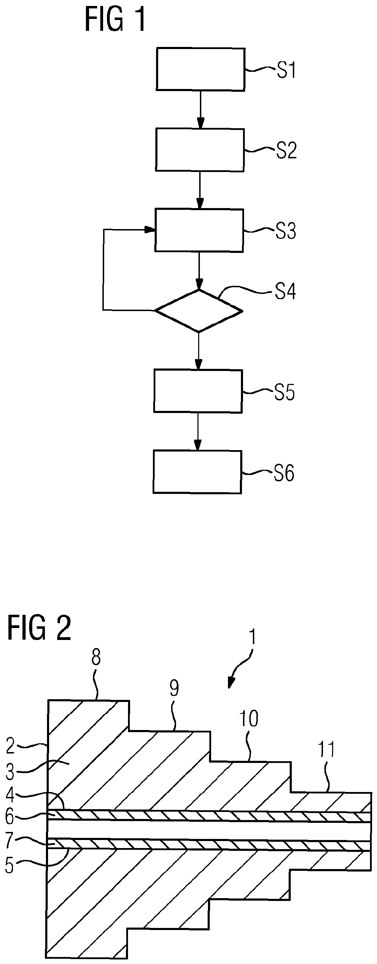

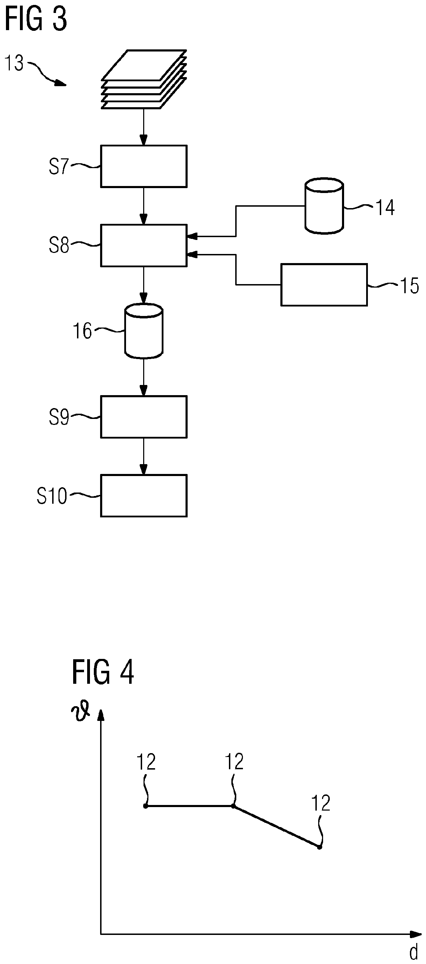

Die Fig. 1 und 3 zeigen Ablaufdiagramme für ein Ausführungsbeispiel des erfindungsgemäßen Verfahrens, wie es zur Vorbereitung einer Strahlentherapieplanung eingesetzt werden kann. Dabei betrifft Fig. 1 zunächst Schritte zur Erzeugung eines Kalibrierungsdatensatzes in einer Kalibrierungsphase, wobei der entsprechende Kalibrierungsdatensatz für eine Mehrzahl folgender, konkreter Untersuchungsvorgänge eines Patienten mit der entsprechenden Röntgeneinrichtung genutzt werden kann, wobei eine entsprechende Nutzung des Kalibrierungsdatensatzes, der in einer Datenbank der Röntgeneinrichtung gespeichert ist, gemäß Fig. 3 näher erläutert wird.

Die in Fig. 1 dargestellte Kalibrierungsphase wird zyklisch wiederholt, beispielsweise in typischen Wartungsintervallen, welche von täglich bis jährlich reichen können. Dabei wird zunächst in einem Schritt S1 ein speziell ausgestaltetes Phantom auf einer Patientenliege der Röntgeneinrichtung seitens eines Bedieners befestigt.

Fig. 2 zeigt eine mögliche Ausgestaltung eines solchen Phantoms 1 genauer. Das Phantom 1 weist einen zylindrischen, in mehreren Durchmessern gestuften Grundkörper 2 aus einem Phantommaterial 3 auf, welches sich bezüglich der Schwächung von Röntgenstrahlung im Wesentlichen äquivalent zu Wasser verhält. Innerhalb des Phantommaterials 3 sind ebenso zylindrisch ausgebildete Kalibrierungsmaterialkörper 4, 5 aus jeweils einem Kalibrierungsmaterial 6, 7 eingebettet, wobei üblicherweise mehr als ein Kalibrierungsmaterial, insbesondere fünf Kalibrierungsmaterialien, verwendet werden, die für eine stöchiometrische Kalibrierung geeignet sind. Beispielsweise kann es sich bei dem Kalibrierungsmaterial 6 um Teflon, bei dem Kalibrierungsmaterial 7 um PMMA handeln.

Der in der Kalibrierungsphase zu erzeugende Kalibrierungsdatensatz soll für unterschiedliche, das von dem Röntgendetektor der Röntgeneinrichtung empfangene Empfangsspektrum beschreibende Spektrumsparameter geeignet seien. Die gestufte Ausführung mit den unterschiedlichen Durchmessern in den Bereichen 8, 9, 10 und 11 des Phantoms 1 zielt dabei auf einen die Ausdehnung eines Patienten beschreibenden Ausdehnungsparameter ab, nachdem als dieser im weiteren Verlauf ein effektiver Durchmesser des Patienten herangezogen werden wird. Nachdem das Phantom 1 vorliegend im Wesentlichen vollständig vermessen werden kann, entfallen unterschiedliche Messungen für unterschiedliche Durchmesser, da diese durch die entsprechende geometrische Ausgestaltung des Phantoms 1 bereits gegeben sind. Nachdem das Phantommaterial 3 wasseräquivalent ist, lassen sich die unterschiedlichen Durchmesser in den Bereichen 8 bis 11 analog zu wasseräquivalenten Durchstrahlungslängen des Patienten verstehen.

Nach der Montage des Phantoms 1 auf der Patientenliege wird in einem Schritt S2 durch den Bediener ein Kalibrierungsprogramm gestartet. Mit dem Start des Kalibrierungsprogramms positioniert die Röntgeneinrichtung selbsttätig das Phantom 1 geeignet und führt in dem Schritt S3 eine erste Kalibrierungsmessung des Phantoms 1 durch, wobei ein Satz bestimmter, vorgegebener röntgeneinrichtungsbezogener Spektrumsparameter verwendet wird, welche das von der Röntgenquelle erzeugte Erzeugungsspektrum beschreibende Erzeugungsparameter, beispielsweise eine Röhrenspannung, und/oder Filterparameter, die verwendete Filter bzw. deren Einstellungen beschreiben, umfassen können. Die Messergebnisse werden zunächst gespeichert.

In einem Schritt S4 wird überprüft, ob noch Messungen für weitere Sätze von röntgeneinrichtungsspezifischen Spektrumsparametern anstehen. Ist dies der Fall, wird für den nächsten Satz von vorgegebenen, zu vermessenden Spektrumsparametern zum Schritt S3 zurückgekehrt, ansonsten, wenn alle Messergebnisse vorliegen, wird mit dem Schritt S5 fortgefahren.

Im Schritt S5 werden die Messergebnisse der Schritte S3 ausgewertet, um einen Kalibrierungsdatensatz zu erhalten. Nachdem vorliegend eine stöchiometrische Kalibrierung verwendet werden soll, wird ein durch freie Modellparameter parametriertes Stöchiometriemodell angesetzt, welches Bildwerte, insbesondere konkret HU-Werte, abhängig von Dichte und stöchiometrischer Zusammensetzung eines Materials mit einem physikalischen Eigenschaftswert verknüpft, mithin eine Zuordnungsvorschrift definiert. Als der eine oder die mehreren physikalischen Eigenschaftswerte können insbesondere eine Elektronendichte, eine Massendichte und ein Bremsvermögen (stopping power) herangezogen werden.

Die freien Modellparameter hängen von den Spektrumsparametern ab. Nachdem die stöchiometrische Zusammensetzung und die entsprechenden Dichten der Kalibrierungsmaterialien 6, 7 bekannt sind und aus den Messergebnissen Bildwerte für verschiedene Spektrumsparameter vorliegen, kann in der Auswertung im Schritt S5 ein Zusammenhang zwischen den Modellparametern und den Spektrumsparametern hergeleitet werden. Dabei können durch Interpolation auch Kalibrierungsdaten bzw. eine komplette Abbildungsvorschrift auch für Werte der Spektrumparameter zwischen den vermessenen Werten erzielt werden, so dass die Kalibrierungsdaten des entstehenden Kalibrierungsdatensatzes, der in einem Schritt S6 dauerhaft in einem Speichermittel der Röntgeneinrichtung gespeichert wird, konkret in einer Datenbank, letztlich die Veränderung des Modells mit verändertem Empfangsspektrum beschreibt.

Fig. 4 zeigt einen beispielhaften Zusammenhang eines Modellparameters υ und eines Spektrumsparameters, hier der wasseräquivalenten Durchstrahlungslänge d, wobei Messpunkte 12 der Kalibrierungsmessung sowie die dazwischenliegende Interpolation angedeutet sind.

Fig. 3 zeigt nun näher, wie der Kalibrierungsdatensatz in einem konkreten Untersuchungsvorgang eines Patienten eingesetzt wird. Nachdem es sich bei der Röntgeneinrichtung vorliegend um eine Computertomographieeinrichtung handelt, liegt der dreidimensionale Bilddatensatz 13 nach der Aufnahme und der Rekonstruktion, wie grundsätzlich bekannt, als Bildstapel von Schnittbildern bzw. Schichtbildern, also für verschiedene Schichten des Patienten, vor. Der Bilddatensatz 13 ist dabei vorliegend als ein DICOM-Datenobjekt vorhanden, so dass auch als Spektrumsparameter verwendete Aufnahmeparameter als Metadaten ebenso in diesem Datenobjekt enthalten sind, beispielsweise das verwendete Erzeugungsspektrum beschreibende Erzeugungsparameter. Um Konvertierungsinformationen im Sinne der stöchiometrischen Kalibrierung zu ermitteln, wird ein einem Schritt S7 nun zunächst durch Auswertung des Bilddatensatzes 13 für jede Schicht der Ausdehnungsparameter des Patienten bestimmt, vorliegend als mittlerer Durchmesser. Alternativ zu einer Ermittlung aus den jeweiligen Schichtbildern des Bildstapels ist es auch möglich, den Ausdehnungsparameter schichtspezifisch aus zur Vorbereitung auf die Aufnahme des Bilddatensatzes 13 aufgenommenen Topogrammen zu ermitteln.

Nachdem der Ausdehnungsparameter als Spektrumsparameter im Schritt S7 ermittelt wurde, liegen mithin alle Spektrumsparameter vor, so dass in einem Schritt S8 schichtspezifisch (aufgrund des schichtspezifischen Ausdehnungsparameters) die Konvertierungsinformation zusammengestellt werden kann, wozu der Kalibrierungsdatensatz 14 und zusätzlich eine Patienteninformation 15 herangezogen wird.

Die Patienteninformation 15 beschreibt beispielsweise den Aufnahmebereich, das Geschlecht und/oder andere physiologische/anatomische Merkmale des Patienten und dient letztlich dazu, durch die Konvertierungsinformation abgedeckte Patientenmaterialien zu ermitteln. So kann beispielsweise die Konvertierungsinformation auf tatsächlich im Aufnahmebereich auftretende Gewebearten beschränkt werden. Die Patienteninformationen 15 können wenigstens teilweise durch die Röntgeneinrichtung selbst ermittelt werden, wenigstens teilweise aber auch durch einen Bediener mittels einer Benutzereingabe bestimmt werden. Insbesondere ist es im Übrigen auch denkbar, ein Benutzerinterface zu verwenden, in dem der Bediener die Dichte und stöchiometrische Zusammensetzung der Patientenmaterialien gezielt konfigurieren kann, um Spezialfällen gerecht zu werden.

Nachdem somit im Schritt S8 dann die Modellparameter für die entsprechenden Spektrumsparameter schichtspezifisch aus den Kalibrierungsdaten des Kalibrierungsdatensatzes 14 bekannt sind bzw. daraus abgeleitet werden können und zudem ermittelt werden konnte, für welche Patientenmaterialien Konvertierungsinformationen benötigt werden, kann die Konvertierungsinformation im Rahmen der üblichen stöchiometrischen Kalibrierung zusammengestellt werden, insbesondere als die zu verwendenden freien Modellparameter und Dichten/stöchiometrische Daten der Patientenmaterialien; möglich ist es aber auch, sogleich die entsprechende Zuordnungsvorschrift, beispielsweise als Look-Up-Tabelle (Konversionstabelle) zu ermitteln und als Konvertierungsinformation zu bestimmen.

Ergebnis ist eine Patienten-, Röntgeneinrichtungs- und aufgrund der Beachtung der wasseräquivalenten Durchstrahlungslänge sogar schichtspezifische Konvertierungsinformation 16, die in einem Schritt S9 als Metadaten dem Bilddatensatz 13 hinzugefügt wird. Dies ist im DICOM-Format, welches hier verwendet wird, problemlos möglich. Das so ergänzte Datenobjekt des Bilddatensatzes 13 wird in einem Schritt S10 von der Röntgeneinrichtung an eine entsprechende Auswertungseinrichtung, hier eine Planungseinrichtung, bereitgestellt. Nachdem der Bilddatensatz 13 in seiner für die Darstellung kontrastoptimierten Form mit den Bildwerten (HU-Werten) noch enthalten ist, kann eine Darstellung zur Bestrahlungsplanung problemlos erfolgen, insbesondere auch unter Lokalisierung des konkreten als Ziel anvisierten Planungsgebiets, beispielsweise eines Tumors. Aufgrund der mitgelieferten Konvertierungsinformation 16 kann jedoch problemlos eine Dosisverteilungsberechnung auf hochgenaue Art und Weise und schnell erfolgen, so dass die Planung insgesamt einfach und unter Bereitstellung eines kompakten Informationspakets stattfinden kann.

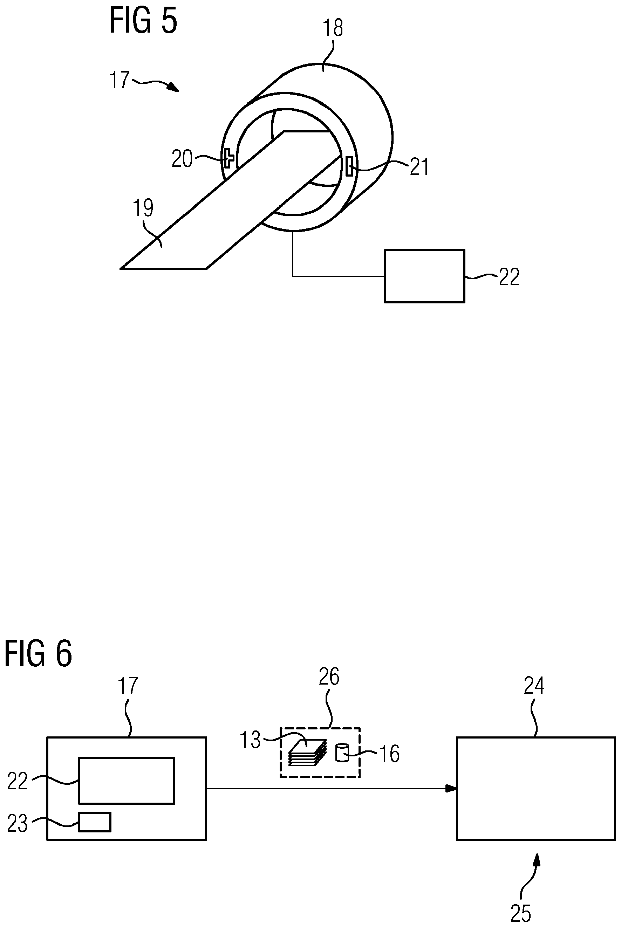

Fig. 5 zeigt eine Prinzipskizze einer erfindungsgemäßen Röntgeneinrichtung 17, hier einer Computertomographieeinrichtung, die entsprechend, wie grundsätzlich bekannt, eine Gantry 18 aufweist, in die ein Patient (oder das Phantom 1) mittels einer Patientenliege 19 eingefahren werden kann. Innerhalb der Gantry ist rotierbar eine Aufnahmeanordnung mit einer Röntgenquelle 20 und einem Röntgendetektor 21 angeordnet, um Projektionsbilder des entsprechenden aufzunehmenden Aufnahmebereichs des Patienten anzufertigen, aus denen aus bekannter Art und Weise Bilddatensätze 13 bzw. entsprechende Schwächungskoeffizienten als Bildwerte ermittelt werden können.

Der Betrieb der Röntgeneinrichtung 17 wird von einer Steuereinrichtung 22 gesteuert, die zur Durchführung des erfindungsgemäßen Verfahrens ausgebildet ist. Das bedeutet, in diesem Fall stellt die Röntgeneinrichtung 17 selbst vollständige DICOM-Datenobjekte mit dem Bilddatensatz 13 und den passenden Konvertierungsinformationen 16 bereit. In alternativen Ausgestaltungen ist es auch möglich, eine Zwischeneinrichtung vorzusehen, die die Konvertierungsinformation 16 ermittelt bzw. eine verteilte Ermittlung der Konvertierungsinformation 16 vorzusehen.

Fig. 6 zeigt ein vollständiges Behandlungssystem zur Therapieplanung, zu dem neben der Röntgeneinrichtung 17 mit der Steuereinrichtung 22 und der hier ebenso angedeuteten Datenbank 23 zur Speicherung des Kalibrierungsdatensatzes 14 auch eine Planungseinrichtung 24 als Auswertungseinrichtung 25 gehört. Das DICOM-Datenobjekt 26 mit dem Bilddatensatz 13 und der Konvertierungsinformation 16 wird der Planungseinrichtung 24 von der Röntgeneinrichtung 17 über eine entsprechende Kommunikationsverbindung bereitgestellt.

Dabei sei an dieser Stelle noch angemerkt, dass allein die vollständige Bereitstellung des um Konvertierungsinformation als Metainformation ergänzten Bilddatensatzes von der Röntgeneinrichtung unmittelbar an eine Auswertungseinrichtung bereits eine vorteilhafte Ausbildung darstellt, die auch unabhängig von der Spektrumsparameterabhängigkeit vorteilhaft eingesetzt werden kann.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Bereitstellung einer eine Zuordnungsvorschrift wenigstens eines physikalischen Eigenschaftswerts eines Materials in einem Voxel zu einem Bildwert dieses Voxels in einem mit einer Röntgeneinrichtung (17) aufgenommenen dreidimensionalen Bilddatensatz (13) beschreibenden Konvertierungsinformation (16), wobei durch Vermessung eines wenigstens ein Kalibrierungsmaterial umfassenden Phantoms (1) in der Röntgeneinrichtung (17) ein Kalibrierungsdatensatz (14) bestimmt wird, der zur Ermittlung der Zuordnungsvorschrift verwendet wird, **dadurch gekennzeichnet, dass** der Bilddatensatz (13) mit einem auf einem Röntgendetektor (21) der Röntgeneinrichtung (17) vermessenen Empfangsspektrum aufgenommen wird, dass durch wenigstens einen Spektrumsparameter beschrieben wird, wobei zur Ermittlung der Zuordnungsvorschrift in Abhängigkeit von dem Spektrumsparameter aus dem verschiedene Empfangsspektren beschreibend gemessenen Kalibrierungsdatensatz (14) abgeleitete Kalibrierungsdaten verwendet werden.

2. Verfahren nach Anspruch 1, **dadurch gekennzeichnet, dass** als Spektrumsparameter ein eine Ausdehnung des Patienten insbesondere schichtspezifisch beschreibender Ausdehnungsparameter verwendet wird und als das Phantom (1) ein ein wasseräquivalent schwächendes Phantommaterial (3) um das wenigstens eine Kalibrierungsmaterial (6, 7) aufweisendes Phantom (1) verwendet wird.

3. Verfahren nach Anspruch 2, **dadurch gekennzeichnet, dass** als das Phantom (1) ein Phantom (1) mit für jedes Kalibrierungsmaterial (6, 7) mehreren geometrischen Ausdehnungen, insbesondere Durchmessern, des das Kalibrierungsmaterial (6, 7) umgebenden Phantommaterials (3) verwendet wird.

4. Verfahren nach Anspruch 2 oder 3, **dadurch gekennzeichnet, dass** der Ausdehnungsparameter einen mittleren Durchmesser des Patienten umfasst, der zur Auswahl der Kalibrierungsdaten als Durchmesser des Phantoms (1) verwendet wird.

5. Verfahren nach einem der Ansprüche 2 bis 4, **dadurch gekennzeichnet, dass** der Ausdehnungsparameter aus dem Bilddatensatz (13) selber und/oder aus einem zur Planung der Aufnahme des Bilddatensatzes (13) aufgenommenen Topogramm ermittelt wird.

6. Verfahren nach einem der Ansprüche 2 bis 5, **dadurch gekennzeichnet, dass** bei für jede Schicht des Bilddatensatzes (13) vorliegendem Ausdehnungsparameter die Konvertierungsinformation (16) schichtspezifisch ermittelt wird.

7. Verfahren nach einem der vorangehenden Ansprüche, **dadurch gekennzeichnet, dass** als Spektrumsparameter wenigstens ein das von einer Röntgenquelle (20) erzeugte Sendespektrum beschreibender Erzeugungsparameter, insbesondere eine Röhrenspannung, und/oder wenigstens ein wenigstens ein verwendetes Filter beschreibender Filterparameter verwendet wird, wobei der Kalibrierungsdatensatz (14) für mehrere unterschiedliche Erzeugungsparameter bzw. Filterparameter vermessen wird.

8. Verfahren nach einem der vorangehenden Ansprüche, **dadurch gekennzeichnet, dass** für wenigstens einen Spektrumsparameter des Bilddatensatzes (13), zu dem keine Kalibrierungsdaten im Kalibrierungsdatensatz (14) vorliegen, passende Kalibrierungsdaten durch Interpolation abgeleitet werden.

9. Verfahren nach einem der vorangehenden Ansprüche, **dadurch gekennzeichnet, dass** eine stöchiometrische Kalibrierung verwendet wird, wobei die freien Modellparameter eines zur Ermittlung der Zuordnungsvorschrift verwendeten Stöchiometriemodells durch die Kalibrierungsdaten abhängig von dem wenigstens einen Spektrumsparameter beschrieben werden.

10. Verfahren nach Anspruch 9, **dadurch gekennzeichnet, dass** die Konvertierungsinformation (16) für mehrere stöchiometrisch beschriebene, innerhalb eines Patienten auftretende Patientenmaterialien ermittelt wird, wobei die Zahl und/oder Art der Patientenmaterialien, für die die Konvertierungsinformation (16) bestimmt wird, aufgrund einer Patienteninformation (15) beschränkt und/oder definiert wird.

11. Verfahren nach Anspruch 10, **dadurch gekennzeichnet, dass** die Patienteninformation (15) wenigstens teilweise automatisch durch die Röntgeneinrichtung (17) und/oder wenigstens teilweise manuell durch eine Benutzereingabe ermittelt wird und/oder einen Aufnahmebereich des Bilddatensatzes (13) und/oder ein verwendetes Aufnahmeprotokoll und/oder das Alter und/oder das Geschlecht des Patienten umfasst.

12. Verfahren nach einem der vorangehenden Ansprüche, **dadurch gekennzeichnet, dass** die Konvertierungsinformation (16) seitens der Röntgeneinrichtung (17) ermittelt und gemeinsam mit dem Bilddatensatz (13) an eine den Bilddatensatz (13) weiter auswertende Auswertungseinrichtung übermittelt wird. .

13. Verfahren nach Anspruch 12, **dadurch gekennzeichnet, dass** die Konvertierungsinformation (16) dem Bilddatensatz (13) als Metadaten hinzugefügt wird, insbesondere als DICOM-Metadaten, und/oder der Bilddatensatz (13) durch Auswertung zur Kontrastoptimierung bezüglich einer Darstellung bearbeitet übermittelt wird.

14. Verfahren nach einem der vorangehenden Ansprüche, **dadurch gekennzeichnet, dass** die Vermessung des Kalibrierungsdatensatzes (14) zyklisch, insbesondere in Wartungsintervallen, wiederholt wird und/oder abgesehen von der Einbringung des Phantoms (1) durch einen Bediener vollständig automatisch nach einem Kalibrierungsprogramm von der Röntgeneinrichtung (17) durchgeführt wird und/oder der insbesondere jeweils aktuelle Kalibrierungsdatensatz (14) in einer Datenbank (23) der Röntgeneinrichtung (17) abgespeichert wird.

15. Röntgeneinrichtung (17), insbesondere Computertomographieeinrichtung, aufweisend eine einen Röntgendetektor (21) und eine Röntgenquelle (20) aufweisende Aufnahmeanordnung und eine zur Durchführung eines Verfahrens nach einem der vorangehenden Ansprüche ausgebildete Steuereinrichtung (22).

16. Computerprogramm, welches die Schritte eines Verfahrens nach einem der Ansprüche 1 bis 14 durchführt, wenn es auf einer Recheneinrichtung ausgeführt wird.

17. Elektronisch lesbarer Datenträger, auf dem ein Computerprogramm nach Anspruch 16 gespeichert ist.

## Claims

1. Method for providing an item of conversion information (16) describing an allocation rule of at least one physical property value of a material in a voxel relating to an image value of this voxel in a three-dimensional image data set (13) recorded with an X-ray apparatus (17), wherein by scanning a phantom (1) comprising at least one calibration material in the X-ray apparatus (17), a calibration database (14) which is used for determining the allocation rule is determined, **characterised in that** the image data set (13) is recorded with a receiving spectrum geared to an X-ray detector (21) of the X-ray apparatus (17), said receiving spectrum being described by at least one spectral parameter, wherein for determining the allocation rule dependent upon the spectral parameter, calibration data derived from the measured calibration data set (14) describing different receiving spectra is used.

2. Method according to claim 1, **characterised in that** as the spectral parameter, an in particular slice-specific extent parameter describing an extent of the patient is used and as the phantom (1), a phantom (1) having a water-equivalent attenuating phantom material (3) round the at least one calibrating material (6, 7) is used.

3. Method according to claim 2, **characterised in that** as the phantom (1), a phantom (1) with for each calibration material (6, 7), a plurality of geometrical extents, in particular diameters, of the phantom material (3) surrounding the calibration material (6, 7) is used.

4. Method according to claim 2 or 3, **characterised in that** the extent parameter comprises a mean diameter of the patient, which is used for selection of the calibration data as the diameter of the phantom (1).

5. Method according to one of claims 2 to 4, **characterised in that** the extent parameter is determined from the image data set (13) itself and/or from a topogram recorded for planning the recording of the image data set (13).

6. Method according to one of claims 2 to 5, **characterised in that** with an extent parameter available for each slice of the image data set (13), the conversion information (16) is determined slice-specifically.

7. Method according to one of the preceding claims, **characterised in that** as a spectral parameter, at least one generating parameter describing the transmitting spectrum generated by an X-ray source (20), in particular a tube voltage and/or at least one filter parameter describing at least one filter that is employed is used, wherein the calibration data set (14) is measured for a plurality of different generating parameters or filter parameters.

8. Method according to one of the preceding claims, **characterised in that** for at least one spectral parameter of the image data set (13) for which no calibration data is available in the calibration data set (14), suitable calibration data is derived by interpolation.

9. Method according to one of the preceding claims, **characterised in that** a stoichiometric calibration is used, wherein the free model parameters of a stoichiometric model used for determining the allocation rule are described by the calibration data dependent upon the at least one spectral parameter.

10. Method according to claim 9, **characterised in that** the conversion information (16) is determined for a plurality of stoichiometrically described patient materials occurring inside a patient, wherein the number and/or type of the patient materials for which the conversion information (16) is determined is restricted and/or defined on the basis of an item of patient information (15).

11. Method according to claim 10, **characterised in that** the patient information (15) is determined at least partially automatically by the X-ray apparatus (17) and/or at least partially manually by a user input and/or comprises a scanning region of the image data set (13) and/or a scanning protocol that is used and/or the age and/or the sex of the patient.

12. Method according to one of the preceding claims, **characterised in that** the conversion information (16) is determined at the X-ray apparatus (17) and is transferred together with the image data set (13) to an evaluating device further evaluating the image data set (13).

13. Method according to claim 12, **characterised in that** the conversion information (16) can be added to the image data set (13) as metadata, in particular as DICOM metadata, and/or the image data set (13) can be transferred processed by evaluation for contrast optimization with regard to a representation.

14. Method according to one of the preceding claims, **characterised in that** the measuring of the calibration data set (14) can be repeated cyclically, particularly in maintenance intervals and/or apart from the introduction of the phantom (1) by an operator, is carried out entirely automatically according to a calibration program by the X-ray apparatus (17) and/or the in particular respective current calibration data set (14) is stored in a database (23) of the X-ray apparatus (17).

15. X-ray apparatus (17), in particular a computed tomography apparatus, having a recording device having an X-ray detector (21) and an X-ray source (20) and a control device (22) configured for carrying out a method according to one of the preceding claims.

16. Computer program which carries out the steps of a method according to one of claims 1 to 14 when said program is executed on a computer unit.

17. Electronically readable data carrier on which a computer program according to claim 16 is stored.

## Revendications

1. Procédé pour disposer d'une information (16) de conversion décrivant une prescription d'association, d'au moins une valeur de propriété physique d'un matériau dans un voxel à une valeur d'image de ce voxel, en un ensemble (13) de données d'image en trois dimensions enregistré par un dispositif (17) à rayons X, dans lequel en mesurant un fantôme (1) comprenant au moins un matériau d'étalonnage dans le dispositif (17) à rayons X, on détermine un ensemble (14) de données d'étalonnage que l'on utilise pour la détermination de la prescription d'association, **caractérisé en ce que** l'on enregistre l'ensemble (13) de données d'image par un spectre de réception, qui est mesuré sur un détecteur (21) de rayons X du dispositif (17) à rayons X et qui est décrit par au moins un paramètre de spectre, dans lequel, pour la détermination de la prescription d'association en fonction du paramètre de spectre, on utilise des données d'étalonnage déduites de l'ensemble (14) de données d'étalonnage mesuré décrivant divers spectres de réception.

2. Procédé suivant la revendication 1, **caractérisé en ce que** l'on utilise, comme paramètre de spectre, un paramètre d'étendue, décrivant une étendue du patient, notamment spécifiquement à une couche et on utilise, comme fantôme (1), un fantôme (1) ayant un matériau (3) fantôme d'atténuation équivalent à l'eau autour du au moins un matériau (6, 7) d'étalonnage.

3. Procédé suivant la revendication 2, **caractérisé en ce que** l'on utilise, comme fantôme (1), un fantôme (1) ayant, pour chaque matériau (6, 7) d'étalonnage, plusieurs étendues géométriques, notamment des diamètres, du matériau (3) fantôme entourant le matériau (6, 7) d'étalonnage.

4. Procédé suivant la revendication 2 ou 3, **caractérisé en ce que** le paramètre d'étendue comprend un diamètre moyen du patient, que l'on utilise comme diamètre du fantôme (1) pour la sélection des données d'étalonnage.

5. Procédé suivant l'une des revendications 2 à 4, **caractérisé en ce que** l'on détermine le paramètre d'étendue à partir de l'ensemble (13) soi même de données d'image et/ou à partir d'un topogramme enregistré pour la planification de l'enregistrement de l'ensemble (13) de données d'image.

6. Procédé suivant l'une des revendications 2 à 5, **caractérisé en ce que** l'on détermine, d'une manière spécifique à une couche, l'information (16) de conversion pour un paramètre d'étendue présent pour chaque couche de l'ensemble (13) de données d'image.

7. Procédé suivant l'une des revendications précédentes, **caractérisé en ce que** l'on utilise, comme paramètre de spectre, au moins un paramètre de production décrivant le spectre d'émission produit par une source (20) de rayons X, notamment une tension de tube et/ou au moins un paramètre de filtre décrivant un filtre utilisé, l'ensemble (14) de données d'étalonnage étant mesuré pour plusieurs paramètres de production et respectivement paramètres de filtre différents.

8. Procédé suivant l'une des revendications précédentes, **caractérisé en ce que**, pour au moins un paramètre de spectre de l'ensemble (13) de données d'image pour lequel il n'y a pas de donnée d'étalonnage dans l'ensemble (14) de données d'étalonnage, on déduit, par interpolation, des données d'étalonnage adaptées.

9. Procédé suivant l'une des revendications précédentes, **caractérisé en ce que** l'on utilise un étalonnage stœchiométrique, dans lequel les paramètres de modèle libres d'un modèle stœchiométrique utilisé pour la détermination de la prescription d'association sont décrits par les données d'étalonnage en fonction du au moins un paramètre de spectre.

10. Procédé suivant la revendication 9, **caractérisé en ce que** l'on détermine l'information (16) de conversion pour plusieurs matériaux de patient décrits stœchiométriquement et se produisant au sein d'un patient, dans lequel on détermine le nombre et/ou le type des matériaux du patient pour lesquels l'information (16) de conversion est limitée et/ou définie sur la base d'une information (15) de patient.

11. Procédé suivant la revendication 10, **caractérisé en ce que** l'on détermine l'information (15) de patient au moins en partie automatiquement par le dispositif (17) à rayons X et/ou au moins en partie manuellement par une entrée d'utilisateur et/ou l'information de patient comprend une partie d'enregistrement de l'ensemble (13) de données d'image et/ou un protocole d'enregistrement utilisé et/ou l'âge et/ou le sexe du patient.

12. Procédé suivant l'une des revendications précédentes, **caractérisé en ce que** l'on détermine l'information (16) de conversion du côté du dispositif (17) à rayons X et on la transmet conjointement avec l'ensemble (13) de données d'image à un dispositif d'analyse analysant davantage l'ensemble (13) de données d'image.

13. Procédé suivant la revendication 12, **caractérisé en ce que** l'on ajoute l'information (16) de conversion à l'ensemble (13) de données d'image comme métadonnées, notamment comme métadonnées DICOM, et/ou **en ce que** l'on transmet l'ensemble (13) de données d'image traité, en ce qui concerne une représentation, par analyse d'optimisation du contraste.

14. Procédé suivant l'une des revendications précédentes, **caractérisé en ce que** l'on répète la mesure de l'ensemble (14) de données d'étalonnage cycliquement, notamment dans des intervalles d'entretien, et/ou l'on effectue, abstraction faite de l'introduction du fantôme (1) par un opérateur, d'une manière entièrement automatique suivant un programme d'étalonnage par le dispositif (17) à rayons X et/ou **en ce que** l'on met, notamment l'ensemble (14) de données d'étalonnage en cours respectivement dans une base (23) de données du dispositif (17) à rayons X.

15. Dispositif (17) à rayons X, notamment dispositif de tomodensitométrie assisté par ordinateur, comportant un agencement d'enregistrement ayant un détecteur (21) de rayons X et une source (20) de rayons X et un dispositif (22) de commande constitué pour effectuer un procédé suivant l'une des revendications précédentes.

16. Programme d'ordinateur, qui effectue les stades d'un procédé suivant l'une des revendications 1 à 14, lorsqu'il est réalisé sur un dispositif informatique.

17. Support de données, déchiffrable électroniquement, sur lequel est mis en mémoire un programme d'ordinateur suivant la revendication 16.
